# EUROPÄISCHE PATENTSCHRIFT

(11) **EP 2 837 353 B1**
(45) Veröffentlichungstag und Bekanntmachung des Hinweises auf die Patenterteilung: **30.09.2015**
(21) Anmeldenummer: 13199128.3
(22) Anmeldetag: 20.12.2013
(51) Int. Cl.: A61B 19/00, B08B 3/12

(54) **Antrieb und Zusatzvorrichtung für Ultraschall-Reinigungsgeräte**
Drive and additional device for ultrasound cleaning devices
Entraînement et dispositif supplémentaire pour appareils de nettoyage à ultrasons

(30) Priorität: 16.08.2013 DE 202013007537 U
(43) Veröffentlichungstag der Anmeldung: 18.02.2015
(73) Patentinhaber: BANDELIN patent GmbH & Co. KG, 12207 Berlin (DE)
(72) Erfinder: Jung, Rainer, 13125 Berlin (DE); Möhricke, Jonas, 10319 Berlin (DE)
(74) Vertreter: Pfenning, Meinig & Partner GbR

(56) Entgegenhaltungen:
- WO-A1-2012/098732
- WO-A1-2012/148266
- US-A- 5 711 921
- US-A1- 2011 132 404

## Beschreibung

Die Erfindung betrifft eine Zusatzvorrichtung für ein Ultraschallgerät bzw. ein Ultraschall-Reinigungsgerät, eine Steuer- und/oder Bedienvorrichtung für ein Ultraschallgerät mit einer derartigen Zusatzvorrichtung, sowie ein Verfahren zur aktiven Bewegung zumindest eines Bereichs eines medizinischen Instruments in einem Ultraschall-Reinigungsgerät.

Bei der Reinigung von medizinischen Instrumenten durch niederfrequenten Ultraschall werden Fremdpartikel auf oder in medizinischen Instrumenten aufgrund spezifischer Ultraschallwirkmechanismen (wie Kavitation, Microstreaming, Jetbuilding, etc.) gelöst.

Hierbei werden insbesondere chirurgische Instrumente in einen Korb gelegt und anschließend in einer mit einer Flüssigkeit befüllten Wanne eines Ultraschall-Reinigungsgeräts eingebracht. Anschließend wird die Wanne mit Ultraschall beaufschlagt, so dass sich Fremdpartikel und Anhaftungen in kurzer Zeit lösen können. Optional wird dieser Prozess durch den Zusatz geeigneter Reinigungs- und/oder Desinfektionspräparate die der Flüssigkeit zugegeben werden unterstützt.

Eine weitere Anwendung ist aus dem Bereich der Laparoskopie bekannt. Hierbei können Fremdpartikel nicht nur an der Außenseite des Instruments, sondern auch in einem Lumen des Schaftes auftreten. Um hier eine reinigende Wirkung zu entfalten, sind Vorrichtungen wie beispielsweise das Produkt Sonomic der BANDELIN electronic GmbH & Co. KG bekannt. Bei diesem Produkt wird an das distale Ende des Schaftes ein Adapter angebracht, welcher es ermöglicht, Flüssigkeit vom proximalen zum distalen Ende des Schaftes zu ziehen, so dass sich die Wirkung des Ultraschalls auch im Inneren des Schaftlumens entfalten kann.

Prinzipiell kann eine derartige Reinigungstechnik auch bei Instrumenten aus dem Bereich der robotergestützten minimal invasiven Chirurgie, wie zum Beispiel die EndoWrist^{®}-Produkte des daVinci-Systems von Intuitive Surgical Inc., angewandt werden. Diese Instrumente weisen zumeist mehr als eine Mechanik auf, wobei eine Mechanik jeweils eine bestimmte Bewegung des Instruments bewirkt, z.B. eine Rotation des Instrumentenschafts, oder eine Bewegung eines Greifers am distalen Ende des Instrumentenschafts.

Die Druckschrift WO 2012/148266 A1 zeigt eine Vorrichtung zu, Reinigen von medizinischen Instrumenten. Die Vorrichtung umfass eine Vielzahl von Adaptern für jeweils ein medizinisches Instrument, wobei der Adapter ein Gehäuse und drei mittels eines Antriebs gegenüber dem Gehäuse bewegbare und mit dem medizinischen Instrument koppelbare Kopplungselemente umfasst, so dass eine Bewegung der Kopplungselemente eine aktive Bewegung eines Bereichs des medizinischen Instruments bewirkt, wobei der Antrieb über ein weiteres Antriebselement verfügt, welches über ein Kraftübertragungselement antreibbar ist, und das weitere Antriebselement über einen linearen Antriebsstrang und auf diesem angeordneten Pins in ein Zahnrad eingreift. Das Zahnrad ist mittels einer Schlupfkupplung mit einer Welle verbunden, welche wiederum mit jeweils einem Koppelelement gekoppelt ist.

Jedoch besteht hier weiterer Bedarf, die Reinigungswirkung für medizinische Instrumente zu verbessern. Hierzu wurde taggleich eine europäische Patentanmeldung der gleichen Anmelderin eingereicht. Aufgabe der vorliegenden Anmeldung ist einen Antrieb für eine Zusatzvorrichtung zur Verfügung zu stellen.

Die Aufgabe wird gelöst gemäß einem Antrieb nach den Merkmalen des Anspruchs 1 sowie einer Zusatzvorrichtung für Ultraschall-Reinigungsgeräte nach den Merkmalen des Anspruchs 14.

Ein Antrieb umfasst zumindest ein Wellenelement, welches über ein erstes Kraftübertragungselement antreibbar ist, wobei das Wellenelement mit mindestens einem Koppelelement über ein zweites Kraftübertragungselement gekoppelt ist, so dass eine Bewegung vom Wellenelement auf das mindestens eine Koppelelement übertragbar ist. Das zweite Kraftübertragungselement ist derart mit dem mindestens einen Koppelelement gekoppelt ist, dass beim Überschreiten eines vorbestimmten Bewegungswiderstands des mindestens einen Koppelelements das zweite Kraftübertragungselement keine Bewegung des Wellenelements auf das mindestens eine Koppelelement überträgt.

Mit Hilfe des vorbeschriebenen Antriebs ist es möglich eine aktive Bewegung von medizinischen Robotikinstrumenten in einem Ultraschallbad zu gewährleisten ohne eine Mechanik zum Bewegen einzelner Teile des Instruments zu beschädigen.

Im Ultraschallbad wird das Instrument derart bewegt bzw. rotiert, dass ein Teilelement des Koppelelements mit der Mechanik des Instruments im Eingriff steht und so eine Bewegung des Koppelelements auf das Instrument übertragen wird. Sobald die Mechanik des Geräts an einem Anschlag angelangt ist, bringt die Mechanik des Instruments einen Bewegungswiderstand auf, welcher nur durch Aufbringen einer sehr hohen Kraft überwunden werden kann. Allerdings würde diese sehr hohe Kraft zu einer Beschädigung der Mechanik des Instruments führen. Der aufgebrachte Bewegungswiderstand der Mechanik des medizinischen Instruments führt zu einem Bewegungswiderstand des Koppelelements, d.h. das Koppelelement durch die Mechanik des Instruments an einer weiteren Drehbewegung gehindert. Der Widerstand zwischen dem zweiten Kraftübertragungselement und dem Koppelelement ist derart gewählt das dieser geringer ist als der von der Mechanik des Instruments bewirkte Bewegungswiderstand. Erreicht eine Mechanik des Instruments also den Anschlag, wird das entsprechende Koppelelement mit einem Bewegungswiderstand beaufschlagt und eine Bewegung des Wellenelements wird nicht mehr auf das Koppelelement übertragen. So kann das Instrument bzw. Teil davon aktiv bewegt werden, die Mechanik des Instruments wird geschützt und das Instrument kann gut gereinigt werden ohne dessen Funktionalität einzuschränken.

In einem weiteren Ausführungsbeispiel sind ein erstes und ein zweites Koppelelemente über jeweils ein zweites Kraftübertragungselement mit einem Wellenelement gekoppelt, so dass sich ein erstes Koppelelement unabhängig vom zweiten Koppelelement bewegen kann. Auf diese Weise ist es nicht wichtig, dass sich die distalen, beweglichen Enden der Instrumente vor dem Einlegen in einen Adapter in einer vorbestimmten Position befinden, da die Bewegungen der Koppelelemente voneinander entkoppelt sind. Für den Fall, das der Antrieb derart ausgebildet ist, dass mehr als ein Koppelelement zur Kopplung an jeweils eine Mechanik des Instruments vorhanden ist, können die Koppelelemente mit einem, vorzugsweise einzigem, Antrieb derart angetrieben werden, dass ein erstes Koppelelement, welches eine erste Mechanik des Instruments an den Anschlag bewegt hat, nicht länger eine Bewegung auf die erste Mechanik überträgt und ein zweites Koppelelements, welches eine zweite Mechanik des Instruments bewegt, diese zweite Mechanik weiterhin in Bewegung hält. In anderen Worten können das erste und ein zweites (oder auch weitere) Koppelelement unabhängig voneinander, jedoch gleichzeitig, eine Bewegung auf eine erste bzw. zweite Mechanik des Instruments übertragen, wobei die mit den Koppelelementen gekoppelten Kraftübertragungselemente gleichzeitig von einem einzigen Antrieb angetrieben werden. Zwischen den Kraftübertragungselementen und den Koppelelementen kann beispielsweise jeweils eine Rutschkupplung angeordnet sein. Gleichzeitig ist hier derart zu verstehen, dass sowohl das erste als auch das zweite Koppelelement zeitgleich jeweils eine mit dem ersten bzw. zweiten Koppelelement gekoppelte erste bzw. zweite Mechanik des Instruments bewegen können. Gelangt eine der ersten oder zweiten Mechanik an einen Anschlag greift eine Rutschkupplung und es wird anschließend nur noch die noch nicht am Anschlag angelangte Mechanik bewegt. Vorzugsweise ist der Antrieb derart konfiguriert, dass mehr als eine Mechanik eines Instruments, und mehr als ein Instrument bewegbar sind, d.h. ein einziger Antrieb zwei oder mehr Instrumente bewegt, wobei die eine oder mehr als eine Mechanik der zwei oder mehr Instrumente gleichzeitig bewegt werden können. Zudem erfolgt die Bewegung der Mechaniken unabhängig voneinander, d.h. während eine am Anschlag angelangte Mechanik nicht weiter bewegt wird, können die weiteren durch den Antrieb und noch nicht an ihrem Anschlag angelangten Mechaniken weiterhin bewegt werden.

Als Kraftübertragungselemente kommen in weiteren Ausführungsformen Riemen, beispielsweise Zahnriemen oder Rundriemen aus Kunststoff, Plastiken oder Gummi zu Einsatz. Weitere Ausführungsformen finden sich in den Ansprüchen.

Mit dem hier beschriebenen Antrieb ist es möglich ein Verfahren zur Steuerung des Antriebs zu implementieren, bei welchem der Antrieb zunächst eine erste Zeitperiode in einer ersten Richtung, und anschließend für eine zweite Zeitperiode in einer zweiten, der ersten Richtung entgegengesetzten Richtung angetrieben wird. Die Länge der Zeitperiode wird unter anderem in Abhängigkeit von der Geschwindigkeit gewählt mit welcher der Antrieb die Koppelelemente antreibt. Da eine Mechanik des Instruments einen maximalen Bewegungsbereich hat, werden die erste und zweite Zeitperiode derart gewählt, dass diese länger ist als die Zeit, in welcher eine durch ein Koppelement angetriebene Mechanik den maximalen Bewegungsbereich durchläuft. Auf diese Weise wird innerhalb eines Zeitraums der ersten und zweiten Zeitperiode erreicht, dass alle durch ein Koppelelement angetriebenen Mechaniken an einem Anschlag angelangt sind. Für den Fall das alle mit jeweils einem Koppelelement gekoppelten Teile einer Mechanik einen identischen Bewegungsbereich (im Sinne einer Distanz), d.h. Bewegungslänge besitzen, kann nach der ersten und zweiten Zeitperiode eine kürzere Zeitperiode eingestellt werden, wobei die kürzere Zeitperiode der Länge entspricht, welche die Teile der Mechaniken benötigen, um den jeweiligen, vollen Bewegungsbereich zu durchlaufen. Für den Fall, dass unterschiedliche Mechaniken unterschiedliche Bewegungsbereiche besitzen, d.h. die durch den Teil der Mechanik zurücklegbare Strecke der Teil sich von einander unterscheiden kann, ist es nach dem durchlaufen der ersten und zweiten Zeitperiode möglich eine kürzere Zeitperiode zu wählen, welche diejenige Mechanik mit der größten zurückzulegenden Strecke für einen vollen Bewegungsbereich benötigt. Hierdurch ist es möglich, dass während der Reinigung der Instrumente in einem Reinigungsbad, insbesondere einem mit Ultraschall beaufschlagten Reinigungsbad, sämtliche Teile der Mechanik über ihren vollen Bewegungsbereich bewegt werden, so dass die Reinigungswirkung der mit dem Ultraschall beaufschlagbaren Flüssigkeit in sämtlichen Bewegungszuständen der durch den Antrieb bewegten Teil der Mechanik wirken kann und so die Reinigungswirkung maximiert wird.

Die erste und zweite Zeitperiode können gleich oder unterschiedlich lang gewählt sein. Da ein an seinem Anschlag angelangter Teil der Mechanik nicht länger durch das Koppelelement bewegt wird (da das Kraftübertragungselement beispielweise durchrutscht bzw. eine Rutschkupplung eine Bewegung des Antriebs auf das Koppelelement entkoppelt), können weitere, durch ein weiteres Koppelelement angetriebenen Teile der Mechanik bis an jeweils ihren Anschlag bewegt werden und dies unabhängig davon, ob andere Teile der Mechanik bereits an ihrem Anschlag angelangt sind. Nach der ersten und zweiten Zeitperiode kann die Länge der folgenden Zeitperioden zur Bewegung des Antriebs in der ersten oder zweiten Richtung reduziert werden, so dass ein Reinigungsdurchlauf insgesamt verkürzt wird ohne die Reinigungswirkung zu reduzieren. Aufwendige Sensorik zur Feststellung, ob eine der bewegten Mechaniken sich bereits am Anschlag befindet, ist nicht notwendig und in einigen Ausführungsbeispielen nicht vorgesehen.

Nachfolgend werden Aspekte der Zusatzvorrichtung beschrieben, welche frei mit den Merkmalen der Ansprüche kombinierbar sind.

Die Zusatzvorrichtung umfasst vorzugsweise ein Gestell und zumindest einen Adapter für ein medizinisches Instrument. Die Zusatzvorrichtung ist derart ausgebildet, dass diese in einer Wanne eines Ultraschallgeräts, vorzugsweise eines Ultraschall-Reinigungsgeräts Platz findet. Dabei ist es vorteilhaft, wenn Teile des Gestells aus der Wanne herausragen, um so eine vereinfachte Handhabung der Zusatzvorrichtung beim Einbringen und Ausbringen aus der Wanne zu gewährleisten.

Der Adapter für das medizinische Instrument umfasst ein Gehäuse und mindestens ein gegenüber dem Gehäuse bewegbares und mit dem medizinischen Instrument koppelbares Kopplungselement. Dieses Kopplungselement ist mittels eines Antriebs bewegbar. Eine Bewegung des Kopplungselements bewirkt bei eingelegtem medizinischem Instrument eine aktive Bewegung zumindest eines Bereichs des medizinischen Instruments.

Instrumente aus dem Bereich der Telemedizin (und weitere Instrumente) besitzen Mechaniken, durch welche es möglich ist, bestimmte Bewegungen des medizinischen Instruments durchzuführen. So kann beispielsweise bei einem endoskopartigen Instrument der Schaft und die Spitze zumindest in einer die Rotationsachse des Schaftes umfassenden Ebene rotiert werden. Weiterhin ist es möglich, dass das Instrument selbst Greifarme oder ähnliches besitzt, welche in einer weiteren Ebene, senkrecht zur vorgenannten Ebene rotierbar ist. Das in dem Adapter angeordnete Kopplungselement ist derart ausgebildet, dass es an eine Mechanik des medizinischen Instruments andocken kann und somit eine Bewegung des Kopplungselements in eine aktive Bewegung zumindest eines Bereichs des medizinischen Instruments umgesetzt wird. Das heißt, das Kopplungselement ist derart ausgebildet, dass eine Bewegung des Kopplungselements, wie beispielsweise eine Rotation des Schaftes des medizinischen Instruments, oder eine Bewegung der distalen Spitze des medizinischen Instrumentes bewirkt wird.

Damit das Kopplungselement zuverlässig an das medizinische Instrument gekoppelt werden kann, so dass eine Bewegung das Kopplungselement in eine aktive Bewegung eines Bereichs des medizinischen Instrumentes umgesetzt wird, kann der Adapter Ausnehmungen, Stege oder ähnliches umfassen, welche zu Stegen bzw. Ausnehmungen eines medizinischen Instruments korrespondieren. Auf diese Weise kann sichergestellt werden, dass das Kopplungselement an vordefinierten Punkten des medizinischen Instruments angreift, vorzugweise an Mechaniken des medizinischen Instruments ansetzt, welche für eine aktive Bewegung zumindest eines Bereichs des medizinischen Instruments notwendig sind.

Aufgrund der aktiven Bewegung des medizinischen Instruments durch die Zusatzvorrichtung, kann das medizinische Instrument während der Beaufschlagung der Wanne mit Ultraschall aktiv bewegt werden, so dass eine verbesserte Reinigungswirkung im Bereich der sich bewegenden Teile des medizinischen Instruments gewährleistet wird. Auf diese Weise wird gewährleistet, dass der Ultraschall auch bei eng aneinander anliegenden mechanischen bzw. beweglichen Teilen des Instruments seine volle Wirkung entfalten kann und Fremdpartikel zuverlässig von dem medizinischen Instrument, auch von verdeckten Teilflächen in z.B. Gelenken, entfernt werden.

In einer Ausführungsform der Zusatzvorrichtung umfasst der Adapter eine Fixierungsvorrichtung zum Fixieren des medizinischen Instruments. Zusätzlich oder optional zu dem bereits erwähnten Ausnehmungen oder Stegen des Adapters, kann eine Fixierungs- oder Verriegelungsvorrichtung vorgesehen sein, welche zumindest einen weiteren Bereich des medizinischen Instruments auf dem Adapter fixiert. Dabei ist die Fixierung dergestalt, dass das Kopplungselement der Zusatzvorrichtung an einem Punkt des medizinischen Instruments angreift, welcher eine aktive Bewegung eines Bereichs des medizinischen Instruments, welcher nicht durch die Fixierungsvorrichtung fixiert ist, bewirkt. Im Falle eines chirurgischen Robotikinstruments kann dies beispielsweise bedeuten, dass das proximale Ende des Instruments, welches zu Zwecken eines medizinischen Eingriffs mit einem Roboterantrieb verbunden wird, in den Adapter eingesetzt wird und durch die Fixiervorrichtung verriegelt wird. Zwar ist nunmehr das proximale Ende des medizinischen Instruments fixiert, jedoch ist das Kopplungselement des Adapters derart an das proximale Ende des medizinischen Instruments gekoppelt, dass sowohl der Schaft als auch die Instrumente am distalen Ende des medizinischen Instruments durch eine Bewegung des Kopplungselements aktiv bewegt werden.

In einer weiteren Ausführungsform umfasst die Zusatzvorrichtung an einer dem medizinischen Instrument zugewandten Oberfläche des Kopplungselements eine Nut oder einen Steg, welche(r) in einen mechanischen Formschluss mit einer Mechanik des zu reinigenden medizinischen Instruments gebracht werden kann. Ist die Mechanik des medizinischen Instruments beispielsweise durch einen Stift bzw. eine Nut gegeben, so ist die Nut bzw. der Steg des Kopplungselements dazu korrespondierend ausgebildet. Auf diese Weise kann eine Bewegung des Kopplungselements direkt auf die Mechanik des medizinischen Instruments übertragen werden.

In einer weiteren Ausführungsform ist das Kopplungselement federnd gelagert. Da das Kopplungselement bevorzugt derart ausgebildet ist, in eine Mechanik des medizinischen Instruments einzugreifen, die Mechanik des medizinischen Instruments und das Kopplungselement jedoch nicht in jeder Orientierung zueinander ineinandergreifen können, kann das Kopplungselement federnd gelagert sein. Dies bewirkt, dass beim Einlegen des medizinischen Instruments das Kopplungselement zunächst nach unten gedrückt wird, sofern es nicht mit der Mechanik des medizinischen Instruments in Eingriff steht, das heißt, dass eine Bewegung des Kopplungselements in eine aktive Bewegung eines Bereichs des medizinischen Instruments umgewandelt werden kann. Durch die federnde Lagerung kann zunächst das Kopplungselement bewegt werden, so dass es eine Position einnimmt, in welcher das Kopplungselement mit der Mechanik des medizinischen Instruments in Eingriff tritt. Dies kann beispielsweise dadurch passieren, dass ein Stift der Mechanik in eine Nut des Adapters eingreift. Erst durch den Eingriff überträgt sich eine Bewegung des Kopplungselements auf die Mechanik und wird so in eine aktive Bewegung des medizinischen Instruments umgewandelt. Durch das Einrutschen des Stiftes in die Nut drückt eine Federwirkung (oder eine vergleichbare Wirkung) das Kopplungselement in Richtung des medizinischen Instruments und bewirkt so einen Formschluss zwischen einer Mechanik des medizinischen Instruments und dem Kopplungselement.

In einer weiteren Ausführungsform ist das Kopplungselement derart durch einen Antrieb bewegbar, dass dieses rotiert wird. Dabei kann der Antrieb derart ausgebildet sein, dass das Kopplungselement abwechselnd in eine und anschießend in eine entgegengesetzte Richtung rotiert wird. Es sind jedoch auch Varianten mit einer linearen Bewegung des Kopplungselements bei entsprechenden medizinischen Instrumenten möglich.

In einer weiteren Ausführungsform der Erfindung ist die Zusatzvorrichtung bzw. das Kopplungselement mechanisch, hydraulisch oder pneumatisch mit einem Antrieb gekoppelt. Hierdurch kann erreicht werden, dass keinerlei elektrische Komponenten des Antriebs sich innerhalb der Wanne des Ultraschallgeräts befinden müssen. Dies vereinfacht die betriebliche Sicherheit und die Zuverlässigkeit der Zusatzvorrichtung. Das Kopplungselement kann demnach mechanische, hydraulische oder pneumatische Getriebe aufweisen, welche eine Verbindung an beispielsweise einen Elektromotor ermöglichen.

In einer weiteren Ausführungsform ist das Gestell derart ausgebildet, dass eine am Gestell angeordnete krafterzeugende Komponente des Antriebs, wie beispielsweise ein Elektromotor außerhalb der Wanne und der mindestens eine Adapter von der krafterzeugenden Komponente beabstandet innerhalb der Wanne angeordnet ist. Hierdurch sind keinerlei elektrische Komponenten der Wirkung des Ultraschalls ausgesetzt, obgleich die medizinischen Instrumente vollständig in der Wanne versenkbar sind. Dies erhöht die Langlebigkeit der Zusatzvorrichtung. Weiterhin kann, beispielsweise im Falle eines Elektromotors, auf weiterführende Sicherheitsmaßnahmen verzichtet werden, da keinerlei elektrische Komponenten in die Wanne bzw. in die mit dem Ultraschall beaufschlagte Flüssigkeit eingebracht wird.

In einer weiteren Ausführungsform ist das Gehäuse des Adapters der Zusatzvorrichtung gegenüber dem Gestell fixiert. Der Adapter ist gegenüber dem Gestell unbeweglich, so dass sich lediglich das Kopplungselement gegenüber dem Gestell bewegen kann. Auf diese Weise ist es möglich, einen Teil des medizinischen Instruments zu fixieren und über das Kopplungselement einen weiteren Bereich des medizinischen Instruments aktiv zu bewegen.

In einer weiteren Ausführungsform umfasst die Zusatzvorrichtung Öffnungen in dem Gehäuse des Adapters, so dass die Flüssigkeit, welche sich in der Wanne befindet, über den Adapter bis in das medizinische Instrument eindringen kann. Hierdurch wird die reinigende Wirkung auf sämtliche Bereiche des medizinischen Instruments verbessert.

In einer weiteren Ausführungsform ist die Zusatzvorrichtung mit einer Saug- und/oder Druckspülungsvorrichtung zur Spülung des medizinischen Instruments verbunden, wobei die Spülungsvorrichtung einen Spüladapter zur Kopplung an das medizinische Instrument umfasst. Zahlreiche Instrumente weisen in einem Schaft ein oder mehrere Lumen auf. Je nachdem, welcher Bereich des medizinischen Instruments in dem Adapter fixiert wird, kann es opportun sein, eine Druckspülung oder eine Saugspülung durch den Schaft vorzunehmen. Hierzu wird eine Saug- und/oder Druckspülungsvorrichtung mittels eines Spüladapters an das medizinische Instrument gekoppelt. In weiteren Ausführungsformen kann alternierend sowohl Flüssigkeit durch den Spüladapter gesogen oder durch diesen in das Instrument gedrückt werden. In weiteren Ausführungsformen ist es möglich, mehr als einen Spüladapter mit dem medizinischen Instrument zu koppeln, um so gleichzeitig in einigen Bereichen eine Saugspülung und in anderen Bereichen des Instruments eine Druckspülung durchzuführen.

Mithilfe der Saug- und/oder Druckspülungsvorrichtung ist es möglich, während der aktiven Bewegung des medizinischen Instruments auch die Lumen des medizinischen Instruments zu spülen, um so die Wirkung des Ultraschalls auch im Inneren der medizinischen Instrumente zu verbessern. Hierbei ist eine Steuerung vorgesehen, welche die aktive Bewegung des medizinischen Instruments, das heißt die Bewegung des Kopplungselements und die Saug- und/oder Druckspülungsvorrichtung steuert.

Ein weiterer Aspekt der Erfindung betrifft eine Steuer- und/oder Bedienvorrichtung für ein Ultraschallgerät, welches zur Steuerung der Zusatzvorrichtung konfiguriert ist. Die Steuerung der Steuer- und/oder Bedienvorrichtung kann dabei eine Steuerungseinheit und eine Speichereinheit umfassen. Die Steuerungseinheit kann beispielsweise programmierbare Field-Arrays, Mikroprozessoren, Mikrocontroller oder ähnliche Vorrichtungen umfassen, welche dazu ausgebildet sind Instruktionen, beispielsweise eines Programmcodes, umzusetzen und hierdurch die Steuerung von Motoren, Pumpen oder ähnlichem zu bewirken. Die Steuer- und/oder Bedienvorrichtung ist dabei derart ausgebildet, dass eine regelmäßige periodische Bewegung des mindestens einen Kopplungselements durch den Antrieb ermöglicht wird. Dies kann beispielsweise bedeuten, dass das Kopplungselement für einen vorbestimmten Zeitraum nach links gedreht oder nach vorne bewegt wird, und für einen weiteren vorbestimmten Zeitraum in die entgegengesetzte Richtung bewegt wird, Die hierzu notwendigen Instruktionen können beispielsweise als Software in der Speichereinheit der Steuer- und/oder Bedienvorrichtung abgelegt werden. Bei der Speichereinheit kann es sich um volatilen oder nicht-volatilen Speicher handeln. Bevorzugt kommt ein Flash-Speicher zur Anwendung.

Weitere Programme zur Bewegung des Kopplungselements können ebenfalls als Software abgelegt werden. Hierdurch kann die jeweils gewünschte Bewegung des Koppelelements bewirkt werden. Die Steuer- und/oder Bedieneinheit kann den Antrieb umfassen oder kann mittels einer Daten- und/oder Stromverbindung mit einem externen, an der Zusatzvorrichtung angeordneten Antrieb verbunden sein. Hierzu ist einer Ausführungsform vorgesehen, dass ein Antrieb der Zusatzvorrichtung mittels eines Kabels und einer Steckverbindung zum einen durch die Steuer- und/oder Bedienvorrichtung mit Strom versorgt wird und zum anderen der Antrieb durch die Steuervorrichtung angesteuert wird.

In einer weiteren Ausführungsform umfasst die Steuer- und/oder Bedienvorrichtung die Spülungsvorrichtung, welche über einen Spüladapter mit dem medizinischen Instrument verbunden werden kann. Dabei ist die Steuer- und/oder Bedienvorrichtung derart konfiguriert, dass diese nicht nur den Bewegungsablauf des Kopplungselements regelt, sondern optional hierzu auch die Spülvorgänge eines Lumens des medizinischen Instrumentes steuert. So kann beispielsweise bei einer Bewegung des Kopplungselements in einer ersten Richtung eine Saugspülung des Instruments und bei dem Bewegen des Kopplungselements in einer zweiten, der ersten entgegengesetzten Richtung eine Druckspülung veranlasst werden. Alternativ kann über eine gleichzeitig aufgebrachte Druck- und -Saugspüfung, welche jeweils über verschiedene Adapter umgesetzt wird, ein Flüssigkeitskreislauf innerhalb der Lumen des medizinischen Instruments erwirkt werden. Der Steuer- und/oder Bedienvorrichtung kommt hierbei die Aufgabe zu, die Spülvorgänge und die aktive Bewegung des medizinischen Instruments aufeinander abzustimmen und somit den größtmöglichen Reinigungseffekt zu erzielen.

In einer weiteren Ausführungsform umfasst die Steuer- und/oder Bedienvorrichtung ein berührungsempfindliches Display. Auf dem berührungsempfindlichen Display können verschiedene Piktogramme für das Abrufen der unterschiedlichen Funktionen der Steuer- und/oder Bedienvorrichtung abgebildet werden. So ist es beispielweise möglich, nur die Spülvorrichtung, nur die aktive Bewegung oder beide Programme gleichzeitig zu starten. Auch die Kontrolle der Ultraschallerzeuger zum Beaufschlagen der Wanne mit Ultraschall wird durch die Steuer- und/oder Bedienvorrichtung durchgeführt. Somit kann die Steuer- und/oder Bedienvorrichtung für verschiedene Ultraschall-Reinigungsgeräte und deren Zubehör eingesetzt werden. Zudem können weitere Funktionalitäten und/oder Ergänzungen der Funktionalitäten durch eine Schnittstelle in die Steuervorrichtung eingespielt werden, so dass Aktualisierungen der Software und einer Erweiterung der Steuerung möglich ist.

Eine Wanne mit Ultraschallwandler zur Beaufschlagung von Instrumenten mit Ultraschall, beispielsweise in einer hier geschilderten Zusatzvorrichtung ist für sich alleine und in Kombination mit der hier beschriebenen Zusatzvorrichtung ein beanspruchbarer Anmeldegegenstand.

Die Ultraschallwandler sind dabei derart an der Wanne angeordnet, dass die an einem Wannenboden angeordneten Ultraschallwandler dem Verlauf eines zu reinigenden Instruments folgend aufgebracht sind. Die Ultraschallwandler können dabei Piezoschallgeber oder andere im Stand der Technik bekannte Ultraschallerzeuger sein. Die Schallwandler werden an der Außenseite der Wanne angeordnet und derart ausgerichtet, dass der Schall im Wesentlichen von der Wanne in das Wanneninnere abgegeben wird.

Hinsichtlich der Anordnung der Ultraschallwandler sind diese im Falle eines sich gerade erstreckenden Instruments derart am Wannenboden platziert, dass mindestens zwei oder mehr Ultraschallwandler genau unterhalb des Robotikinstruments liegen. Für den Fall, dass die Zusatzvorrichtung mehr als ein Instrument fixiert, werden die Ultraschallwandler beispielsweise entlang mehrerer Reihen angeordnet, wobei eine Reihe jeweils genau unterhalb einem eingelegten Instrument angeordnet ist. Es kann vorgesehen sein an der Zusatzvorrichtung Abstandsvorrichtungen anzubringen, um die Zusatzvorrichtung gegenüber der Wanne genau auszurichten bzw. zu positionieren. Für den Fall, dass die zu reinigenden Instrumente gekrümmt sind, werden die Ultraschallwandler am Wannenboden ebenfalls dem Verlauf des Instruments folgend angeordnet. D.h. die geometrische Anordnung der Ultraschallwandler orientiert sich an der Geometrie des zu reinigenden Instruments. Bevorzugt wird zumindest ein Ultraschallwandler derart platziert, dass dieser genau unterhalb einer zu reinigenden distalen Einheit eines Robotikinstruments angeordnet ist. Auf diese Weise wird die Reinigung der Instrumente gegenüber herkömmlichen Ultraschallwandleranordnungen verbessert.

In einer Ausführungsform umfasst die Wanne auch mindestens einen Ultraschallwandler an einer Wannenwand. Dieser ist beispielsweise derart angeordnet, dass dieser im Bereich etwaiger Spülanschlüsse eines zu reinigenden Instruments angeordnet ist. Außer Robotikinstrumenten können auch andere chirurgische Instrumente, wie z.B. Instrumente für die MIC (Minimal-Invasive Chirurgie) in die Wanne eingesetzt werden.

Weitere Ausführungsbeispiele werden anhand der nachfolgenden Figuren erläutert.

Es zeigen:
- FIG. 1: eine Aufsicht eines Ultraschall-Reinigungsgerätes mit einer Zusatzvorrichtung;
- FIG. 2: Detailansicht der Zusatzvorrichtung der FIG. 1;
- FIG. 3a: weitere Detailansicht der Zusatzvorrichtung der FIG. 1;
- FIG. 3b: Beispiel einer Fixiervorrichtung eines Adapters; und
- FIG. 4: Steuerungseinheit und Spülvorrichtung einer geöffneten Steuer- und/oder Bedieneinheit;
- Fig. 5a: eine Variante eines Antriebs für eine Zusatzvorrichtung;
- Fig. 5b: der Antrieb der Fig. 5a aus einer geänderten Perspektive;
- Fig. 5c: Detailansicht von Komponenten der Fig. 5a;
- Fig. 6a, b: Beispiele einer Anordnung von Schallwandlern zur Beaufschlagung von Instrumenten mit Ultraschall.

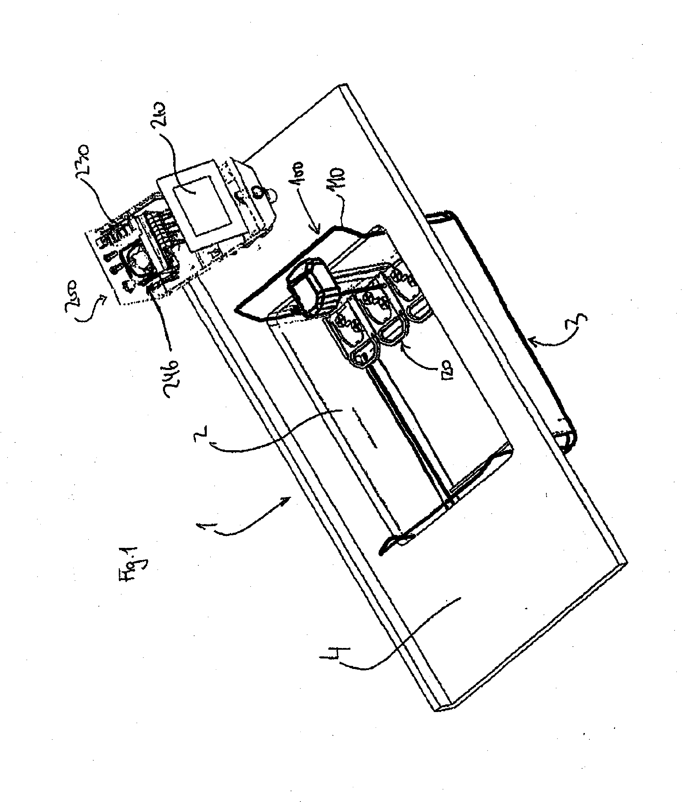

Die FIG. 1 zeigt ein Ultraschall-Reinigungsgerät 1, welches eine Wanne 2 umfasst, die mit einer Flüssigkeit befüllt werden kann. Auf der Unterseite 3 der Wanne befinden sich Schallübertragungselemente (nicht dargestellt), welche Ultraschall auf die Innenwände der Wanne 2 übertragen und so Ultraschallwellen in die in der Wanne 2 befindliche Flüssigkeit übertragen. Bei dem dargestellten Ultraschall-Reinigungsgerät handelt es sich um ein Einbaugerät, welches in die Oberfläche einer Ablage 4 eingelassen ist.

In der Wanne 2 befindet sich eine Zusatzvorrichtung 100, welche in den nachfolgenden Figuren näher erläutert wird. Die Zusatzvorrichtung 100 umfasst ein Gestell 110, welches über die Oberkante der Wanne 2 hinausragt. Am unteren Ende des Gestells 110 befindet sich eine Adapterbank 120, welche derart angeordnet ist, dass diese in die Wanne 2 eingelassen werden kann. Ist die Wanne 2 nun mit Flüssigkeit befüllt, bedeckt die Flüssigkeit die Adapterbank 120 und etwaige darauf angeordnete medizinische instrumente vollständig.

Die Steuer- und/oder Bedieneinheit 200 kann über eine Kabelverbindung mit einem Antrieb der Zusatzvorrichtung verbunden werden. Über das Kabel wird sowohl ein Motor des Antriebs mit Strom versorgt, als auch angesteuert, so dass dieser Bewegungen ausführt. Die Steuerung der Steuer- und Bedieneinheit 200 durch einen Benutzer wird über das berührungsempfindliche Display 210 vorgenommen. Es kann sich dabei um einen kapazitiven oder resistiven berührungsempfindlichen Bildschirm handeln. Weiterhin ist in der Darstellung der FIG. 1 eine Pumpe 246 zu erkennen. Weiterhin ist eine Steuerungsplatine 230 mit darauf angeordneten Prozessoren und Speichereinheiten dargestellt.

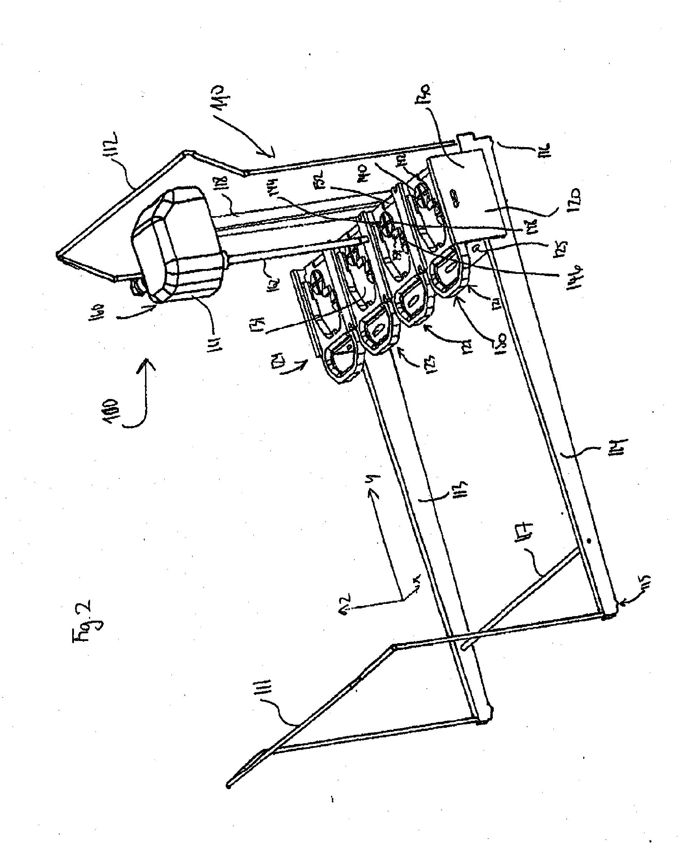

In der FIG. 2 ist die Zusatzvorrichtung 100 ohne die sie in der FIG. 1 umgebenden Wanne 2 dargestellt. Das Gestell 110 umfasst zwei in der x-z-Ebene verlaufende Griffteile 111 und 112, mittels derer die Zusatzvorrichtung 100 aus und In die Wanne gestellt werden kann. Am unteren Ende der Griffe 111 und 112 befinden sich Schienen 113 und 114, welche die beiden Griffe miteinander verbinden. An einem Ende der Schienen 113 und 114 ist zudem eine Adapterbank 120 angeordnet, welche im vorliegenden Ausführungsbeispiel vier Adapter 121 bis 124 umfasst.

Das Gestell 110 kann aus Metall oder einem Kunststoff gefertigt sein. Auch ist es möglich, dass beispielsweise die Griffe 111 und 112 aus Metall und die Schienen 113 und 114 aus einem Kunststoff gefertigt werden. An den Schienen 113, 114 befinden sich zudem Erhebungen 115 und 116, so dass das Gestell nur punktweise mit der Wanne in Berührung ist. Die konstante Breite des Gestells wird über Querstreben 117 gewährleistet.

Die Adapterbank 120 umfasst ein Gehäuse 130, welches zugleich das Gehäuse für die vier Adapter 121 bis 124 bildet. Das Gehäuse ist aus Kunststoff gefertigt und umfasst Öffnungen 125 welche es erlauben, dass in der Wanne befindliche Flüssigkeit ins Innere des Gehäuses 130 eindringen kann.

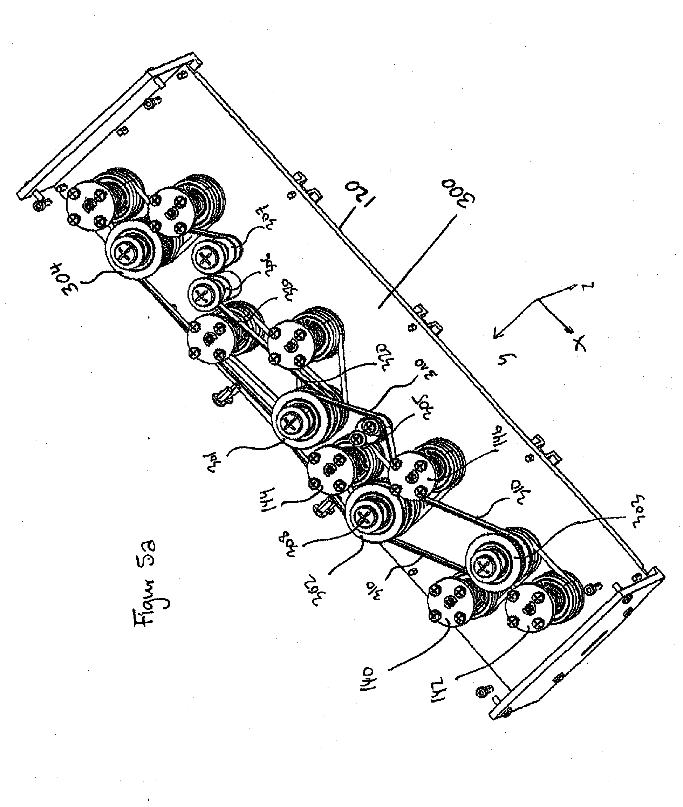

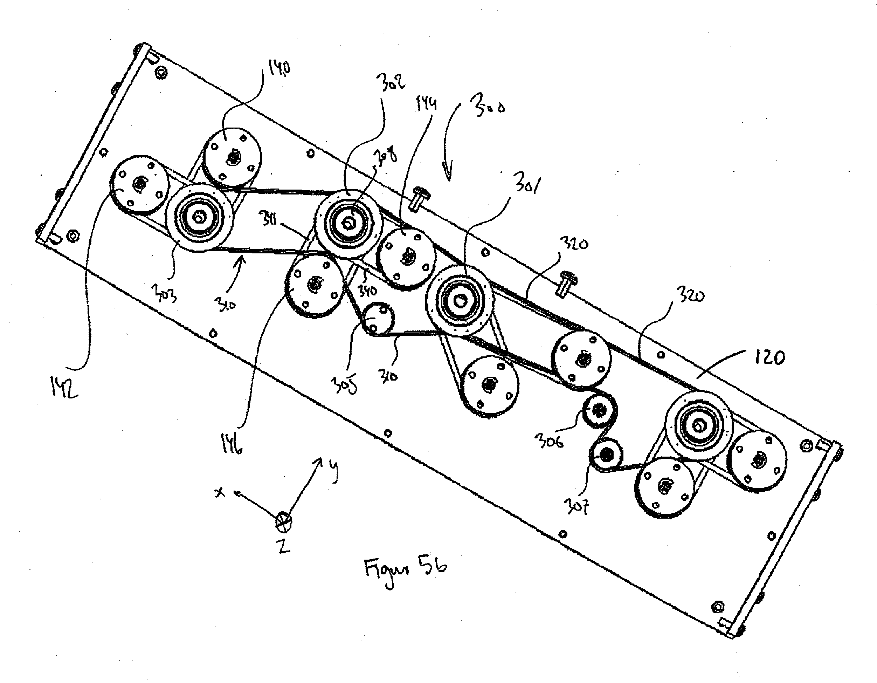

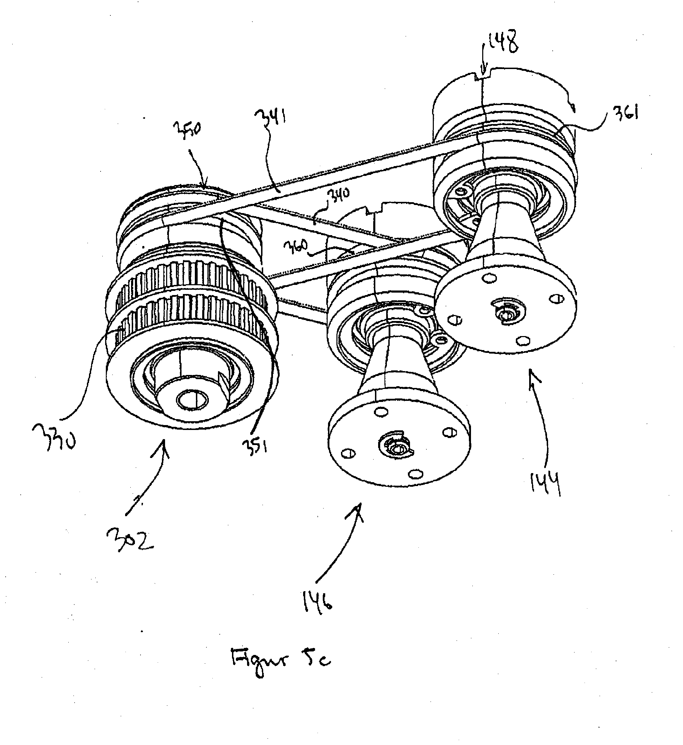

Der Adapter 121 umfasst mindestens ein gegenüber dem Gehäuse bewegliches Kopplungselement 140 bzw. 142. Die Kopplungselemente können aus Kunststoff, Edelstahl, Titan, anderen medizinischen Werkstoffen oder anderen chemisch beständigen bzw. wasserbeständigen Werkstoffen gefertigt sein und stehen über die Oberfläche 131 des Gehäuses 130 in z-Richtung hinaus. Die beweglichen Kopplungselemente 140 und 142 sind in der z-Richtung federnd gelagert. Die Kopplungselemente sitzen auf einer in der Darstellung der FIG. 2 nicht erkennbaren Welle, welche über eine Mechanik, eine Hydraulik oder eine Pneumatik angetrieben wird. Die Welle ist an einem Boden des Gehäuses verankert und weist einen Federmechanismus auf, welche eine Federung des Kopplungselementes in z-Richtung erlaubt. Das heißt, das Kopplungselement kann zumindest um die Höhe, in der es über die Oberfläche 131 hinaussteht, in negativer z-Richtung eingedrückt werden. Hierdurch ist es möglich, dass das Kopplungselement nachgibt und die Drehung des Kopplungselements soweit voranschreitet, dass ein an einem medizinischen Instrument angeordneter Stift in die Nut des Kopplungselements eingreift und somit ein Formschluss erzeugt wird, so dass die Bewegung des Kopplungselements in eine aktive Bewegung des medizinischen Instruments umgesetzt wird. Die medizinischen Instrumente werden zudem mit einer Fixiervorrichtung 150 an ihrem proximalen Ende in der Adapterbank 120 fixiert. Hierdurch ist eine genaue Ausrichtung zwischen dem Kopplungselementen und der Mechanik der medizinischen Instrumente gegeben. An der Zusatzvorrichtung 100 ist zudem ein Antrieb 160 angeordnet, welcher am oberen Ende im Bereich des Griffs 112 angeordnet ist. Dieser umfasst ein Gehäuse 161, in welchem sich in diesem Falle ein Elektromotor befindet. An dem Elektromotor befindet sich eine Koppelstange 162, welche durch eine Öffnung in die Adapterbank 120 geführt wird und dort über das mechanische, pneumatische oder hydraulische Getriebe 300 mit den einzelnen Kopplungselementen gekoppelt ist (siehe Figuren 5). Auf diese Weise kann eine Drehbewegung des Antriebs 160 unter anderem in eine Drehbewegung der Kopplungselemente umgesetzt werden. Das Gehäuse 161 ist im vorliegenden Beispiel über eine Strebe 118 mit der Adapterbank verbunden.

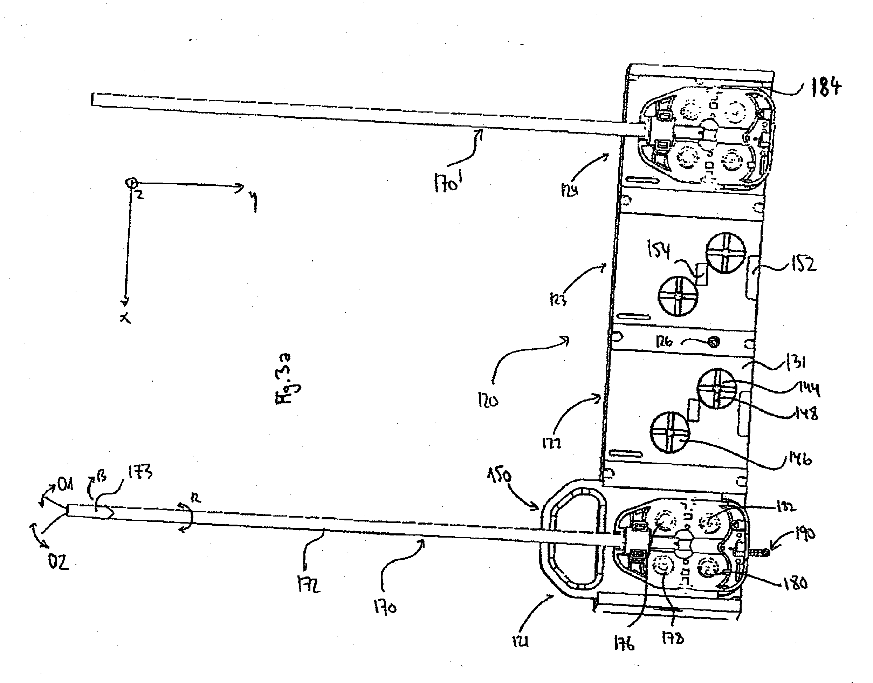

In der FIG. 3a ist eine Aufsicht auf die Adapterbank der FIG. 1 und 2 in der x-y-Ebene dargestellt. Neben der Adapterbank 120 sind eine Fixiervorrichtung 150, als auch zwei medizinische Instrumente 170 und 170' zu sehen, so dass die Interaktion zwischen dem Instrument 170 und den Kopplungselementen genauer erläutert werden kann. Das Instrument 170 umfasst einen Schaft 172, welcher entlang der Achse des Schaftes in der Richtung R rotierbar ist. Der Schaft 172 umfasst an seinem distalen Ende zudem eine bewegliche Spitze 173, welche in der y-z-Ebene in Richtung B rotierbar ist. An der Spitze 173 befinden sich zwei Arme einer Klemme 174, welche jeweils individuell geöffnet und geschlossen werden können, wobei ein Arm jeweils in der x-y-Ebene in einer Richtung O1 bzw. 02 bewegt werden. An seinem proximalen Ende umfasst das Instrument ein (in dieser Aufsicht aufgeschnittenes) Gehäuse, welches unter anderem vier Mechaniken 176, 178, 180 und 182 umfasst. Dabei bewirkt eine Rotation des Elements 176 ein Öffnen in der Richtung 02, die Mechanik 180 eine Rotation in Richtung O1, das Element 178 eine Rotation in der Richtung R und das Element 182 eine Rotation in der Richtung B. In dem hier dargestellten Ausführungsbeispiel umfasst jeder der Adapter der Adapterbank 120 zwei bewegliche Kopplungselemente 144 und 146, welche jeweils zwei kreuzförmig zueinander verlaufenden Nuten 148 umfasst. Die Nuten sind dabei derart gewählt, dass ein an der nicht dargestellten Unterseite der Mechaniken 176 bis 182 befindlicher Stift in den Nuten formschlüssig gehalten werden kann. Sobald der Stift in der Nut 148 formschlüssig gehalten ist, wird eine Bewegung des Kopplungselements 144 beispielsweise in eine Bewegung der Mechanik 182 und somit eine aktive Bewegung der Spitze 173 in der Richtung B umgesetzt. Die Mechaniken können jeweils über einen Bewegungsbereich, d.h. eine bestimmte Länge hinweg verdreht werden, um von einem ersten an einen zweiten Anschlag zu gelangen. Der erste und zweite Anschlag entspricht den Extrempositionen desjenigen Teils des Instruments welches durch die Mechanik bewegt wird.

Wie bereits zur FIG. 2 erwähnt, stehen die Kopplungselemente 140, 142, 144 und 146 in der z-Richtung über die Oberfläche 131 leicht hinaus. Wird nun das medizinische Instrument 170 in dem Adapter eingelegt, drücken die Stifte an der Unterseite der Mechanik des medizinischen Instruments zunächst auf den Bereich des Kopplungselements 144 bzw. 146, welcher die Nuten begrenzt. Hierdurch wird das Kopplungselement in z-Richtung nach unten gedrückt. Bei einer Drehung des Kopplungselements rutscht der Stift der Mechanik nun auf der die Nuten 148 begrenzenden Oberfläche entlang, bis dieser in eine der Nuten eingreift. Hierdurch ist der Druck auf das federnd gelagerte Kopplungselement 144 reduziert und das Kopplungselement wird in positiver z-Richtung nach oben gedrückt. Hierdurch wird der Formschluss zwischen der Nut und dem Stift des medizinischen Instruments herbeigeführt.

Obgleich in dem hier gezeigten Ausführungsbeispiel lediglich zwei Kopplungselemente je medizinischem Instrument vorhanden sind, können in weiteren Ausführungsformen auch weniger, das heißt eins, oder mehr Kopplungselemente, das heißt drei bis vier, vorhanden sein, um sämtliche Bewegungen des distalen Bereichs des medizinischen Instruments aktiv herbeizuführen. Dabei kann die Bewegung des distalen Teils des medizinischen Instruments dergestalt sein, dass das Kopplungselement 144 zunächst um bestimmten im Uhrzeigersinn und anschließend um gleichen Winkel gegen den Uhrzeigersinn bewegt wird. Hierdurch wird eine aktive Bewegung auf das medizinische Instrument übertragen, so dass die distale Spitze 173 über einen Winkelbereich von beispielsweise 180° abgeknickt werden kann und die Arme der Klemme über einen Bereich von ebenfalls nahezu 180° bewegt werden können.

Die Nuten 148 dienen unter anderem auch der Fixierung des medizinischen Instruments. In anderen Ausführungsbeispielen können auch Löcher oder Stege die Funktion der Nuten übernehmen. Die Form des Fixierungselements, welches am Kopplungselement angeordnet ist, hängt somit im Wesentlichen von den Mechaniken der medizinischen Instrumente ab. Besitzen die medizinischen Instrumente Stifte, so sind Nuten oder Löcher zu bevorzugen. Besitzen diese Löcher oder Nuten, werden Stege oder Zapfen auf dem beweglichen Kopplungselement verwendet.

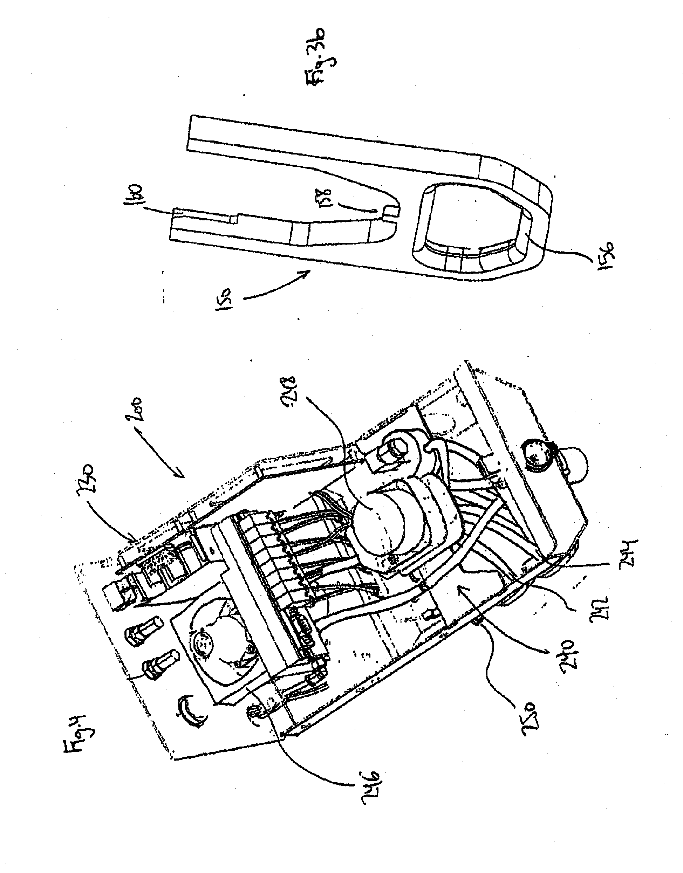

Neben den Nuten weist jeder Adapter noch weitere Fixierhilfen auf. So ist beispielsweise eine Aussparung 152 vorhanden, welche in die Oberfläche 131 eingelassen ist. In diesem Bereich befinden sich bei dem dargestellten medizinischen Instrument elektrische Kontakte, welche über die Unterseite des medizinischen Instruments leicht herausragen. Diese können in der Aussparung 152 ausgerichtet werden, so dass die Positionierung des medizinischen Instruments im Adapter vereinfacht wird. Desweiteren ist ein Steg 154 vorhanden, welcher ebenfalls in eine korrespondierende Aussparung der Unterseite des medizinischen Instruments eingreift. Die Fixiervorrichtung 150 weist zudem wie in der FIG. 3b erkennbar, eine Rastnase 158 auf, welche in eine korrespondierende Aussparung des medizinischen Instruments eingreift. Zudem umfasst die Fixiervorrichtung 150 einen Hinterschnitt 160, welcher mit einem Vorsprung 184 des medizinischen Instruments formschlüssig abschließt. Auf diese Weise ist das im Adapter 121 fixierte medizinische Instrument 170 in seiner Ausrichtung gegenüber dem Adapter 121 fixiert. Lediglich die Kopplung über das Kopplungselement 144 bzw. 146 und die entsprechende Mechanik 178 bzw. 182 bewirkt eine aktive Bewegung des distalen Endes des medizinischen Instruments. Weitere Teile oder Bereiche des medizinischen Instruments werden nicht durch die Kopplungselemente bewegt.

Die Fixiervorrichtung 150 umfasst zudem einen Griff 156, welcher in negativer γ-Richtung gezogen, das medizinische Instrument 170 freigibt. Dieser Zustand ist beispielsweise im Adapter 124 zu erkennen. In weiteren Ausführungsbeispielen umfasst das Fixierungselement 150 einen Anschlag, so dass dieses nur, beispielsweise, über die halbe Länge des Adapters aus diesem gezogen werden kann und anschließend in dem Adapter verbleibt. Auch in dieser Position ist ein Lösen des medizinischen Instruments vom Adapter möglich. Sowohl das Adaptergehäuse 130 als auch die Kopplungselement 144, 146 und der nicht näher dargestellte Getriebestrang, welcher im Gehäuse 130 sitzt, können aus Plastik oder Metall gefertigt sein. Die Fixiervorrichtung 150 kann ebenfalls aus Kunststoff oder Metall gefertigt werden.

In der FIG. 4 ist die Steuer- und/oder Bedienvorrichtung 200 ohne eine obere Abdeckung und das berührungsempfindliche Display 210 dargestellt. Neben der Steuerplatine 230 ist eine Spülvorrichtung 240 erkennbar, welche Schläuche 242/244 für eine Saugspülung und/oder eine Druckspülung umfasst. Die Schläuche bzw. die darin sich befindende Spülflüssigkeit wird mittels einer Pumpe 246 angesogen bzw. gedrückt. Die Schläuche 242 bzw. 244 können beispielsweise mit dem in der FIG. 3a dargestellten Spüladapter 190 gekoppelt werden (der mit dem medizinischen Instrument 170 verbunden ist), welcher das Lumen des Schaftes 172 mit dem Schlauch verbindet. Anschließend kann über das berührungsempfindliche Display ein Programm initiiert werden, welches eine Druck- bzw. Saugspülung des Lumen des Schaftes 172 ermöglicht.

Die Steuerung 230 ist derart programmiert, dass die einzige Pumpe 246 alle vier sich in der Adapterbank 120 befindlichen Instrumente mit einer Saug- bzw. Druckspülung beaufschlagen kann. Hierbei wird das Saugen bzw. Drücken kanalweise, das heißt ein Adapter nach dem anderen, durchgeführt, so dass zumindest für die Saugspülung festgestellt werden kann, ob das Lumen des Schaftes des medizinischen Instruments verstopft ist oder nicht. Die Umschaltung zwischen Saug- und Druckbetrieb wird durch einen Saug-/und Druckumschalter 248 durchgeführt. Die Kanalumschaltung, d.h. die kanalweise Beaufschlagung findet in einem nicht dargestellten Kanalumschalter statt. Die Verstopfung kann dabei über den durch die Pumpe tretenden Fluss bzw. den damit verbundenen Druck ermittelt werden. Diese Technik kommt teilweise auch in den Produkten Sonomic zum Einsatz.

Die Steuervorrichtung 230 umfasst, wie eingangs erwähnt, einen Prozessor und eine Speichereinheit. In der Speichereinheit sind verschiedene Programme zur Bewegung der Instrumente abgespeichert. So kann abhängig vom eingelegten Instrument ein Programm gewählt werden, welches den entsprechenden Winkelbereich der Drehung des Instruments und die anzusteuernden Kopplungselemente definiert. Diese Programme werden über den Prozessor initialisiert und der Prozessor übernimmt eine Steuerung des Antriebs, so dass dieser gemäß dem Programm die Bewegung der Kopplungselemente einleitet.

In der FIG. 4 ist ebenfalls ein Anschluss 250 erkennbar, über welchen eine Verbindung zwischen dem Antrieb 160 der Zusatzvorrichtung 100 mit der Bedien- und/oder Steuereinheit 200 stattfinden kann. Bei einer Verbindung kann es sich beispielsweise um eine Kupfer-Verbindung handeln.

In der Fig. 5a sind in dem Gehäuse 120 angeordnete Komponenten des Antriebs 160 näher dargestellt. Die dargestellte Perspektive ist von der Unterseite des Gehäuses in z-Richtung blickend. Es ist eine Mechanik 300 zu erkennen, welche unter anderem vier Wellenelemente 301-304 umfasst. Dabei ist das Wellenelement 301 direkt mit der Koppelstange 162 verbunden, so dass eine Bewegung des im Gehäuse 161 angeordneten Motors direkt auf das Wellenelement 301 übertragen wird.

Das Wellenelement 301 ist mittels eines ersten Kraftübertragungselements 310 in Form eines Zahnriemens mit den Wellenelementen 302 und 303 gekoppelt, so dass eine Drehbewegung des Wellenelements 301 nach links eine korrespondierende Bewegung der Wellenelemente 302 und 303 bewirkt. Zusätzlich wird der Zahnriemen ein Umlenkelement 305 geführt.

Das Wellenelement 301 ist des Weiteren über ein weiteres erstes Kraftübertragungselement 320 mit dem Wellenelement 304 gekoppelt. Auch dieses Kraftübertragungselement 320 wird über Umlenkelemente 306 und 307 geführt.

Die ersten Kraftübertragungselemente 310 und 320 sind im vorliegenden Beispiel als Zahnriemen ausgebildet, welche in entsprechende Zähne 330 der Wellenelemente 301-304 eingreifen. Zudem stehen die Zahnriemen unter einer mechanischen Spannung, welche die Bewegungsübertragung der Wellenelemente untereinander weiter begünstigt.

In weiteren Ausführungsformen kann das erste Kraftübertragungselement auch ein Riemen ohne Zähne sein. In weiteren Ausführungsformen kann das Kraftübertragungselement eine hydraulische oder pneumatische Bewegungsübertragung vorsehen. So können die Zähne der Wellenelemente beispielsweise ähnlich Turbinenschaufeln ausgebildet sein, so dass durch das Wellenelement 301 ohne einen (geschlossenen) Kanal bewegte Flüssigkeit oder Luft eine Bewegung der weiteren Wellenelemente 302-304 bewirkt.

Die Wellenelemente sind zylinderförmig ausgebildet und sind auf einer Welle drehbar gelagert. Im Falle des Wellenelements 302 ist die Welle 308 erkennbar. Als Lager kann beispielsweise eine Gleit- oder Kugellager vorgesehen sein.

Jedes Wellenelement 301-304 ist mit jeweils zwei Koppelelementen gekoppelt. So ist beispielsweise das Wellenelement 302 mit den Koppelelementen 144 und 146 über jeweils ein zweites Kraftübertragungselement 340 und 341 in Form jeweils eines Rundriemens gekoppelt. Dies ist insbesondere in der Figur 5b deutlich erkennbar. Dabei ragen die mit den Nuten 148 versehenen Oberflächen der Koppelelemente 144 und 148 aus Öffnungen des Gehäuses 120 heraus wie dies beispielsweise in Fig. 2 dargestellt ist. Die Wellenelemente ragen nicht durch eigene Öffnungen aus der Oberfläche heraus.

Der Rundriemen läuft in einer Ausnehmung des Wellenelements als auch des Koppelelements. Dies ist beispielsweise in der Figur 5c dargestellt. Das zweite Kraftübertragungselement 340 läuft in einer Rille 350 des Wellenelements und einer Rille 360 des Koppelelements. Das zweite Kraftübertragungselement 341 läuft in einer Rille 351 des Wellenelements und einer Rille 361 des Koppelelements. Wird nun das Wellenelement entweder direkt über die Koppelstange 162 oder über ein erstes Kraftübertragungselement in Drehbewegung versetzt, überträgt sich die Drehbewegung des Wellenelements über die zweiten Kraftübertragungselemente auf die Koppelelemente.

Wie vorab beschrieben, kann eine Mechanik eines medizinischen Robotikinstruments in beispielsweise die Nut 148 eingreifen (bzw. eine korrespondierende Nut eines anderen Koppelelements), Auf diese Weise wird eine Bewegung des Motors, über die Koppelstange 162, die Wellenelemente 301-304 und die zweiten Kraftübertragungselemente auf die Koppelelemente übertragen und die distalen Enden der beispielsweise Instrumente 170 bzw. 170' werden bewegt bzw. der Schaft des Instruments rotiert.

Im Falle das das Instrument derart bewegt bzw. rotiert wurde, dass die Mechanik des Geräts an einem Anschlag angelangt ist, bringt die Mechanik des Instruments einen Bewegungswiderstand auf, welcher nur durch Aufbringen eine sehr hohen Kraft überwunden werden kann. Allerdings würde diese sehr hohe Kraft zu einer Beschädigung der Mechanik des Instruments führen. Der aufgebrachte Bewegungswiderstand der Mechanik des medizinischen Instruments führt zu einem Bewegungswiderstand des Koppelelements, d.h. das Koppelelement wird aufgrund der in die Nut 148 eingreifenden Mechanik an einer weiteren Drehbewegung gehindert. Der Reibungswiderstand zwischen dem zweiten Kraftübertragungselement und dem Koppelelement ist derart gewählt das dieser geringer ist als der von der Mechanik des Instruments bewirkte Bewegungswiderstand. Von daher rutscht der Rundriemen in der Rille und das Koppelelement führt keine weitere Bewegung aus. Da im vorliegenden Ausführungsbeispiel ein erstes und eine zweites Koppelelemente über jeweils ein zweites Kraftübertragungselement mit einem Wellenelement gekoppelt sind, kann sich ein erstes Koppelelement unabhängig vom zweiten Koppelelement bewegen in dem Sinne, dass eine Durchrutschen des Rundriemens des ersten Koppelelements keinen Einfluss auf eine etwaige Bewegung des zweiten Koppelelements hat. Auf diese Weise ist es nicht wichtig, dass sich die distalen, beweglichen Enden der Instrumente vor dem Einlegen in einen Adapter in einer vorbestimmten Position befinden. Wird der Motor in jeweils eine Drehrichtung für eine Strecke gedreht, welche dem vollen Drehumfang der Mechanik des Instruments entspricht, bzw. dem vollen maximalen Drehumfang der verschiedenen Mechaniken (jeweils zum Bewegen eines unterschiedlichen Teils des Instruments, wie z.B. der Rotation des Schaftes oder dem Rotieren des distalen Endes oder verschiedener in verschiedene Adapter der Adapterbank eingelegte Instrumente) entspricht, kann sichergestellt werden, dass alle in einen Adapter eingelegten Instrumente über ihren vollständigen Bewegungsumfang bewegt werden, unabhängig von der Ausgangsstellung der Mechaniken der Instrumente. Dabei werden die Mechaniken zeitweise gleichzeitig bewegt, d.h. nicht nacheinander bewegt, solange die durch das Koppelelement bewegte Mechanik noch nicht am Anschlag angelangt ist. Erreicht eine Mechanik den Anschlag, wird das entsprechende Koppelelement mit einem Bewegungswiderstand beaufschlagt und eine Bewegung des Wellenelements wird nicht mehr auf das Koppelelement übertragen, das im vorliegenden Ausführungsbeispiel der Rundriemen durchrutscht. Weiterhin werden jedoch Mechaniken, welche noch nicht am Anschlag angelangt sind, weiterbewegt. Außer Rundriemen sind weitere Riemen als zweite Kraftübertragungselemente einsetzbar, wie beispielsweise Flachriemen.

Nachdem der Motor in eine erste Richtung gedreht hat, wird eine Drehbewegung in entgegengesetzter Richtung über zumindest die vorhergehende Strecke durchgeführt. Ab diesem Zeitpunkt kann eine Synchronisation der Bewegungen der eingelegten Instrumente gewährleistet sein. Die Dauer der ersten Drehung und der darauffolgenden zweiten Drehung, d.h. die erste und zweite Zeitperiode sind derart gewählt, dass diese länger als die Zeit, welche eine der bewegten Mechaniken bei der gegebenen oder eingestellten Geschwindigkeit des Antriebs benötigt um seinen jeweiligen vollen Bewegungsbereich zu durchlaufen. Optional, kann anschließend die Dauer der Bewegung in der ersten oder zweiten Richtung reduziert werden.

Bei der Auslegung der Reibungskraft des Rundriemens gegenüber dem Koppelelement sollte berücksichtigt werden, dass sich die Zusatzvorrichtung während der Reinigung in einer Flüssigkeit befindet, welche in das Gehäuse 120 eindringen kann und somit den Bewegungswiderstand, ab welchem eine Durchrutschen möglich ist, heben oder absenken kann.

Im Falle einer hydraulischen oder pneumatischen Lösung kann ein Kanal, welcher eine Kopplung des Wellenelements mit dem Koppelelement bewirkt ein Überdruckventil umfassen. Bringt nun eine Mechanik eines Instruments einen Bewegungswiderstand auf das Koppelelement auf, erhöht sich der Druck im Kanal und das Ventil öffnet sich beim Übersteigen eines Drucks.

In dem dargestellten Ausführungsbeispiel führt somit die Bewegung des Wellenelements nicht länger zu einer Bewegung des Koppelelements, da der auf das Koppelelement aufgebrachte Bewegungswiderstand zu hoch ist und einen vorbestimmten Bewegungswiderstand überschreitet. Der vorbestimmte Bewegungswiderstand ist dabei derart gewählt, dass eine Mechanik eines zu reinigenden Instruments nicht beschädigt wird.

In der Figur 5c ist weiterhin erkennbar, dass ein Wellenelement zwei Zahnreihen für jeweils einen Zahnriemen bzw. zwei Rillen für jeweils einen Rundriemen umfasst. In anderen Ausführungsbeispielen kann die Anzahl jeweils variiert werden.

Alternativ zu dem Merkmal, dass sich die Bewegung des Wellenelements nicht auf das Koppelelement überträgt, wird in dieser Anmeldung auch verstanden, dass das Koppelelement eine funktionale Zweiteilung aufweisen kann. So kann vorgesehen sein, dass ein oberer, mit dem Instrument in Eingriff bringbarer Teil des Koppelelements mit einem zweiten, mit dem zweiten Kraftübertragungselement gekoppelten Teil des Koppelelements derart verbunden ist, dass bei Beaufschlagung des oberen Teils des Koppelelements mit einem Bewegungswiderstand eine Bewegungsentkopplung des oberen und unteren Teil des Koppelelements erfolgt, im Sinne, dass die beiden Teil weiterhin miteinander verbunden sind (beispielsweise über eine Lagerung der Welle), jedoch unabhängig voneinander verdreht werden können, sobald der obere Teil einen Bewegungswiderstand erfährt. Der obere und untere Teil können beispielsweise jeweils zueinander korrespondierende Erhebungen und Vertiefungen umfassen (z.B.: Wellenmuster), welche einen Formschluss in der Richtung der Kraftübertragung aufweisen, wobei der Formschluss durch Aufbringen des vorbestimmten Bewegungswiderstands aufhebbar ist und die beiden Teile gegeneinander rotiert werden können. Hierzu sollten der obere und der untere Teil gegeneinander höhenverstellbar sein, beispielsweise entlang einer Lagerwelle. Der obere und der untere Teil sind also über eine Kupplung miteinander verbunden, wobei die Kupplung einen Leerlauf umfasst, so dass bei Beaufschlagung des oberen Teils mit einem Bewegungswiderstand die Kupplung in den Leerlauf übergeht und eine Bewegung des oberen Teil vom unteren Teil entkoppelt ist.

Weiterhin kann das Merkmal, dass sich die Bewegung des Wellenelements nicht auf das Koppelelement überträgt, auch dadurch ausdrücken lassen, dass eine Kupplung zwischen dem zweiten Kraftübertragungselement und dem Koppelelement vorhanden ist, welche durch das Beaufschlagen des Koppelelements mit einem Bewegungswiderstand in einen Leerlauf übergeht.

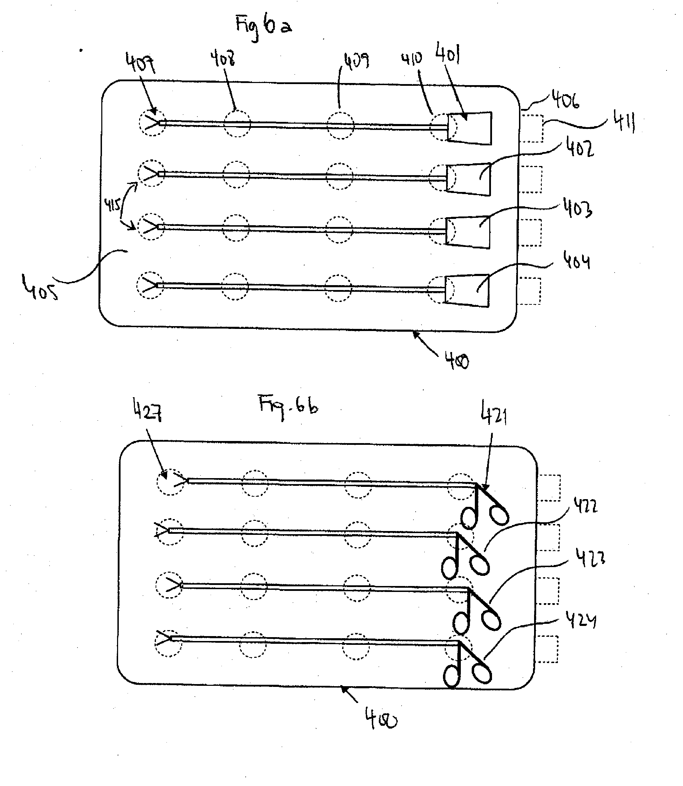

In den Figuren 6a und 6b sind zwei Wannen zur Ultraschallreinigung dargestellt.

In der Fig. 6a ist eine Wanne 400 gezeigt, in welcher eine nicht eingezeichnete Zusatzvorrichtung zum Fixieren von medizinischen Instrumenten mit vier eingelegten Instrumenten 401-404 angeordnet ist. Beispielsweise kann es sich bei der Zusatzvorrichtung um die in dieser Anmeldung beschriebene Zusatzvorrichtung 100 handeln. Die Position der Instrumente 401-404 ist durch die Zusatzvorrichtung vorgegeben.

Am Wannenboden 405 und an der Wannenwand 406 sind Ultraschallwandler 407-411 eingezeichnet. Da die gewählte Aufsicht auf die Wanne von Außen ins Wanneninnere ist, sind die an der Außenseite des Wannenbodens und der Wannenwand angeordneten Ultraschallwandler schraffiert dargestellt, da sie aus der gewählten Perspektive eigentlich nicht sichtbar sind. Dabei befinden sich die Ultraschallwandler 407-410 am Wannenboden und der Ultraschallwandler 411 an der Wannenwand.

Der Ultraschallwandler 407 ist genau unterhalb der distalen Spitze bzw, in der unmittelbaren Nähe der distalen Spitze des Instruments 401 angeordnet. Die Ultraschallwandler 408 und 409 sind unterhalb des Schaftes des Instruments angeordnet. Der Ultraschallwandler 410 befindet sich unterhalb des Adapters 412 des Instruments. Der Adapter wird des Weiteren durch den an der Wannenwand angeordneten Ultraschallwandler 411 beschallt, wobei dieser auf der Höhe des Instruments, vorzugsweise auf der Höhe der Spülanschlüsse des Instruments angeordnet ist. Unterhalb bzw. an der Seite der weiteren Instrumente 402-404 befinden sich weitere Ultraschallwandler 413, welche analog zu den Ultraschallwandlern 407-411 angeordnet sind. Der Abstand der Ultraschallwandler 407-410 untereinander kann gleichmäßig sein.

In der Fig. 6b ist die Wanne 400 mit einer (nicht dargestellten) alternativen Zusatzvorrichtung zur Halterung von MIC Instrumenten 421-424 dargestellt. Die Anordnung der Ultraschallwandler ist auch hier derart, dass zumindest ein Ultraschallwandler 427 im Bereich der distalen Spitze des Instruments angeordnet ist.

## Patentansprüche

1. Zusatzvorrichtung (100) für eine Wanne (2) eines Ultraschallgeräts (1), welche ein Gestell (110) und mindestens einen Adapter (121-124) für ein medizinisches Instrument aufweist, wobei der Adapter ein Gehäuse (130) und mindestens ein mittels eines Antriebs (160) gegenüber dem Gehäuse bewegbares und mit dem medizinischen Instrument koppelbares Kopplungselement (140, 142; 144, 146) umfasst, so dass eine Bewegung des Kopplungselements eine aktive Bewegung zumindest eines Bereich des medizinischen instruments bewirkt, wobei der Antrieb zumindest ein Wellenelement umfasst, welches über ein erstes Kraftübertragungselement antreibbar ist, und das Wellenelement mit dem mindestens einem Koppelelement über ein zweites Kraftübertragungselement gekoppelt ist, so dass eine Bewegung vom Wellenelement auf das mindestens eine Koppelelement übertragbar ist, wobei das zweite Kraftübertragungselement derart mit dem mindestens einen Koppelelement gekoppelt ist, dass beim Überschreiten eines vorbestimmten Bewegungswiderstands des mindestens einen Koppelelements das zweite Kraftübertragungselement keine Bewegung des Wellenelements auf das mindestens eine Koppelelement überträgt und wobei ein Adapter zwei oder mehr, mit jeweils einem zweiten Kraftübertragungselement verbundene Koppelelemente umfasst, wobei der Antrieb derart konfiguriert ist, dass die jeweils zweiten Kraftübertragungselemente gleichzeitig durch den Antrieb bewegbar sind.

2. Zusatzvorrichtung nach einem der vorhergehenden Ansprüche, wobei der Adapter eine bewegbare Fixierungsvorrichtung (150) zum Fixieren des medizinischen Instruments (170) umfasst.

3. Zusatzvorrichtung nach einem der vorhergehenden Ansprüche, wobei das Kopplungselement an einer dem medizinisches Instrument zugewandten Oberfläche eine Nut (148) zur Kopplung an das medizinische Instrument umfasst.

4. Zusatzvorrichtung nach einem der vorhergehenden Ansprüche, wobei das Kopplungselement mechanisch, hydraulisch oder pneumatisch mit dem Antrieb gekoppelt ist.

5. Zusatzvorrichtung nach einem der vorhergehenden Ansprüche, das Gestell derart ausgebildet ist, dass eine krafterzeugende Komponente des Antriebs außerhalb der Wanne und der mindestens eine Adapter innerhalb der Wanne angeordnet ist.

6. Zusatzvorrichtung nach einem der vorhergehenden Ansprüche, wobei eine Saug- und/oder Druckspülungsvorrichtung zur Spülung des medizinischen Instruments vorhanden ist, wobei die Spülungsvorrichtung (240) einen Spüladapter (190) zur Kopplung an das medizinische Instrument und/oder einen Saug- und Druckumschalter (248) umfasst.

7. Verfahren zur aktiven Bewegung zumindest eines Bereich eines medizinischen Instruments in einer Wanne eines Ultraschallbads, wobei das medizinische Instrument in einen Adapter eingelegt wird und derart mit zwei oder mehreren gegenüber einem Gehäuse beweglichen Kopplungselementen des Adapters gekoppelt wird, wobei jeweils ein Kopplungselement an jeweils eine Mechanik des Instruments koppelt, so dass eine Bewegung der zwei oder mehr Kopplungselemente eine aktive, gleichzeitige Bewegung der jeweils gekoppelten Mechaniken des medizinischen Instruments bewirkt und eine Bewegung eines Antriebs von einem Koppelelement entkoppelt wird, wenn eine mit dem Koppelelement gekoppelte Mechanik an einem Anschlag angelangt ist.

8. Verfahren nach Anspruch 7, wobei das medizinische Instrument während der Beaufschlagung der Wanne mit Ultraschall bewegt wird.

9. Verfahren nach einem der Ansprüche 7 oder 8, wobei während der aktiven Bewegung des medizinischen Instruments eine Kavität des medizinischen Instruments eine Flüssigkeit des Ultraschallbads in die Kavität gesogen und/oder gedrückt wird.

10. Verfahren nach einem der Ansprüche 7 bis 9, wobei der Antrieb für die Dauer einer erste Zeitperiode in eine erste Richtung und anschließend für eine zweite Zeitperiode in eine zweite, der ersten Richtung entgegengesetzten Richtung gedreht wird, wobei die erste und zweite Zeitperiode derart gewählt ist, dass diese länger andauert als die Zeit in welcher ein voller Bewegungsbereich der jeweils gekoppelten Mechaniken durchlaufen wird.

11. System umfassend eine Zusatzvorrichtung nach einem der vorhergehenden Ansprüche 1 bis 6 und eine Steuer- und/oder Bedienvorrichtung (200) für ein Ultraschallgerät (1) und zur Steuerung der Zusatzvorrichtung (100), wobei die Steuer- und/oder Bedienvorrichtung derart konfiguriert ist, dass eine Bewegung des mindestens einen Kopplungselement durch den Antrieb bewirkt wird.

12. System nach Anspruch 11, wobei die Steuer- und/oder Bedienvorrichtung eine Spülvorrichtung (240) mit einem Saug- und Druckumschalter (248) aufweist.

13. System nach Anspruch 11 oder 12, wobei die Steuer- und/oder Bedienvorrichtung derart konfiguriert ist, dass der Antrieb eine erste Zeitperiode in einer ersten Richtung bewegt wird und anschließend eine zweite Zeitperiode in einer zweiten, der ersten Richtung entgegengesetzten Richtung bewegt wird, wobei die erste und zweite Zeitperiode derart gewählt sind, dass diese länger sind als eine Zeitperiode welche zum Durchlaufen eines vollen Bewegungsbereichs jeweils einer Mechanik des Instruments notwendig sind.

14. System nach Anspruch 13, wobei die Bewegung in der ersten und zweiten Richtung regelmäßig aufeinanderfolgend über die erste bzw. zweite Zeitperiode erfolgt.

## Claims

1. An auxiliary device (100) for a tank (2) of an ultrasound apparatus (11), which comprises a rack (110) and at least one adapter (121-124) for a medical instrument, wherein the adapter comprises a housing (130) and at least one coupling element (140, 142; 144, 146) which is movable with respect to the housing by way of a drive (160) and which can be coupled to the medical instrument, so that a movement of the coupling element effects an active movement of at least one region of the medical instrument, wherein the drive comprises at least one shaft element which can be driven via a first force transmission element, and the shaft element is coupled to the at least one coupling element via a second force transmission element, so that a movement can be transmitted from the shaft element onto the at least one coupling element, wherein the second force transmission element is coupled to the at least one coupling element in a manner such that on exceeding a predefined movement resistance of the at least one coupling element, the second force transmission element transmits no movement of the shaft element onto the at least one coupling element, and wherein an adapter comprises two or more coupling elements which are connected in each case to a second force transmission element, wherein the drive is configured in a manner such that the respective second force transmission elements are simultaneously movable by the drive.

2. An auxiliary device according to any one of the preceding claims, wherein the adapter comprises a movable fixation device (150) for fixing the medical instrument (170).

3. An auxiliary device according to any one of the preceding claims, wherein the coupling element on a surface facing the medical instrument comprises a groove (148) for coupling onto the medical instrument.

4. An auxiliary device according to any one of the preceding claims, wherein the coupling element is mechanically, hydraulically or pneumatically coupled to the drive.

5. An auxiliary device according to any one of the preceding claims, wherein the rack is designed in a manner such that a force-producing component of the drive is arranged outside of the tank, and the at least one adapter within the tank.

6. An auxiliary device according to any one of the preceding claims, wherein a suction and/or a pressure rinsing device is present for rinsing the medical instrument, wherein the rinsing device (240) comprises a rinsing adapter (190) for coupling onto the medical instrument and/or onto a suction and pressure change-over switch (248).

7. A method for the active movement of at least one region of a medical instrument in a tank of an ultrasound bath, wherein the medical instrument is applied into an adapter and is coupled to two or more coupling elements of the adapter, which is movable with respect to the housing, and wherein each of the coupling elements is coupled to a respective mechanism of the medical instrument, in a manner such that a movement of the two or more coupling elements effects an active simultaneous movement of the respectively coupled mechanism of the medical instrument and a movement of a drive is decoupled from a coupling element when a mechanism coupled with said coupling element reaches a stop.

8. A method according to claim 7, wherein the medical instrument is moved during the subjection of the tank to ultrasound.

9. A method according to claim 7 or 8, wherein a fluid of the ultrasound bath is sucked and/or pressed into a cavity of the medical instrument during the active movement of the medical instrument.

10. A method according to any one of claims 7 through 9, wherein the drive for the duration of the first time period is rotated in a first direction and subsequently for a second time period in a second direction which is opposite to the first direction, wherein the first and the second time period is selected in a manner such that this lasts longer than the time in which a complete movement region of the respective coupled mechanisms is run through.

11. System comprising an auxiliary device according to one of claims 1 through 6 and a control and/or operating device (200) for an ultrasound apparatus (1) and for the control of the auxiliary device (100), wherein the control and/or operating device is configured such that a movement of the at least one coupling element is effected by the drive.

12. System according to claim 11, wherein the control and/or operating device comprises a rinsing device (240) with a suction and pressure change-over switch (248).

13. System according to claim 11 or 12, wherein the control and/or operating device is configured in a manner such that the drive for a first time period is moved in a first direction and subsequently for a second time period in a second direction which is opposite to the first direction, wherein the first and the second time period are selected in a manner such that these are longer than a time period which is necessary for running through a complete movement region in each case of a mechanism of the instrument.

14. System according to claim 13, wherein the movement in the first and second direction is effected regularly in a consecutive manner over the first and the second time period respectively.

## Revendications

1. Dispositif supplémentaire (100) pour un bac (2) d'un appareil à ultrasons (1), qui comprend un châssis (110) et au moins un adaptateur (121-124) pour un instrument médical, l'adaptateur comprenant un boîtier (130) et au moins un élément de couplage (140, 142 ; 144, 146) pouvant être déplacé par rapport au boîtier à l'aide d'un dispositif d'entraînement (160) et pouvant être couplé avec l'instrument médical, de façon à ce qu'un mouvement de l'élément de couplage provoque un mouvement actif d'au moins une partie de l'instrument médical, le dispositif d'entraînement comprenant au moins un élément d'arbre, qui peut être entraîné par l'intermédiaire d'un premier élément de transmission de force, et l'élément d'arbre étant couplé avec l'au moins un élément de couplage par l'intermédiaire d'un deuxième élément de transmission de force, de façon à ce qu'un mouvement de l'élément d'arbre puisse être transmis à l'au moins un élément de couplage, le deuxième élément de transmission de force étant couplé avec l'au moins un élément de couplage, de façon à ce que, lors d'un dépassement d'une résistance au mouvement prédéterminée de l'au moins un élément de couplage, le deuxième élément de transmission de force ne transmet aucun mouvement de l'élément d'arbre à l'au moins un élément de couplage et un adaptateur comprenant deux éléments de couplage ou plus, reliés chacun avec un deuxième élément de transmission de force, le dispositif d'entraînement étant conçu de façon à ce que les deuxièmes éléments de transmission de force puissent être déplacés simultanément par le dispositif d'entraînement.

2. Dispositif supplémentaire selon l'une des revendications précédentes, l'adaptateur comprenant un dispositif de fixation mobile (150) pour la fixation de l'instrument médical (170).

3. Dispositif supplémentaire selon l'une des revendications précédentes, l'élément de couplage présentant, au niveau d'une surface du dessus orientée vers l'instrument médical, un orifice (148) pour le couplage à l'instrument médical.

4. Dispositif supplémentaire selon l'une des revendications précédentes, l'élément de couplage étant couplé de manière mécanique, hydraulique ou pneumatique avec le dispositif d'entraînement.

5. Dispositif supplémentaire selon l'une des revendications précédentes, le châssis étant conçu de façon à ce qu'une composante d'application de force du dispositif d'entraînement soit disposée à l'extérieur du bac et l'au moins un adaptateur soit disposé à l'intérieur du bac.

6. Dispositif supplémentaire selon l'une des revendications précédentes, un dispositif d'aspiration et/ou de rinçage sous pression est prévu pour le rinçage de l'instrument médical, le dispositif de rinçage (240) comprenant un adaptateur de rinçage (190) pour le couplage à l'instrument médical et/ou à un commutateur d'aspiration et de pression (248).

7. Procédé de déplacement actif d'au moins une partie d'un instrument médical dans un bac d'un bain à ultrasons, l'instrument médical étant inséré dans un adaptateur et étant couplé avec deux éléments de couplage de l'adaptateur ou plus, mobiles par rapport à un boîtier, chaque élément de couplage couplant un mécanisme de l'instrument, de façon à ce qu'un mouvement des deux éléments de couplage ou plus provoque le mouvement simultané des dispositifs mécaniques couplés de l'instrument médical et un mouvement d'un dispositif d'entraînement est découplé d'un élément de couplage lorsqu'un mécanisme couplé à l'élément de couplage a atteint une butée.

8. Procédé selon la revendication 7, l'instrument médical étant déplacé pendant l'application d'ultrasons dans le bac.

9. Procédé selon l'une des revendications 7 ou 8, un liquide à ultrasons étant aspirée et/ou comprimée vers une cavité de l'instrument médical pendant le mouvement actif de l'instrument médical.

10. Procédé selon l'une des revendications 7 à 9, le dispositif d'entraînement étant tourné pendant la durée d'une première période dans une première direction puis pendant une deuxième période dans une deuxième direction, opposée à la première direction, la première et la deuxième période étant choisies de façon à ce qu'elles soient plus longues que le temps pendant lequel une aire de mouvement complète de chacun des mécanismes couplés est parcourue.

11. Système comprenant un dispositif supplémentaire selon l'une des revendications précédentes 1 à 6 et un dispositif de commande (200) pour un appareil à ultrasons (1) et pour la commande du dispositif supplémentaire (100), le dispositif de commande étant conçu de façon à ce qu'un mouvement de l'au moins un élément de couplage est provoqué par le dispositif d'entraînement.

12. Système selon la revendication 11, le dispositif de commande (240) comprenant un commutateur d'aspiration et de pression (248).

13. Système selon la revendication 11 ou 12, le dispositif de commande étant conçu de façon à ce que le dispositif d'entraînement étant déplacé, pendant une première période, dans une première direction puis, pendant une deuxième période, dans une deuxième direction, opposée à la première direction, la première et la deuxième période étant choisies de façon à ce qu'elles soient plus longues qu'une période nécessaire pour le parcours d'une aire de mouvement complète d'un mécanisme de l'instrument.

14. Système selon la revendication 13, les mouvements dans la première et la deuxième direction se succédant régulièrement pendant la première ou la deuxième période.
